# EUROPEAN PATENT SPECIFICATION

(11) **EP 1 276 463 B1**
(45) Date of publication and mention of the grant of the patent: **15.12.2010**
(21) Application number: 01930907.9
(22) Date of filing: 27.04.2001
(51) Int. Cl.: A61K 9/00, A61K 31/14, A61K 33/00, A61P 7/04

(54) **HEMOSTATIC AGENT, METHOD AND CARRIER FOR APPLYING A BLOOD CLOTTING AGENT**
HÄMOSTATISCHE WIRKSUBSTANZ, METHODE UND TRÄGER ZUR VERABREICHUNG EINER BLUTGERINNENDEN WIRKSUBSTANZ
AGENT HEMOSTATIQUE, ET PROCEDE ET VECTEUR D'APPLICATION D'UN AGENT DE COAGULATION DU SANG

(30) Priority: 28.04.2000 US 200207 P; 13.06.2000 US 592344; 19.01.2001 US 766513
(43) Date of publication of application: 22.01.2003
(73) Proprietor: Biolife, L.L.C., Sarasota, FL 34243 (US)
(72) Inventor: PATTERSON, James, A., Sarasota, FL 34234 (US); THOMPSON, John, A., Nassau (BS); KEENE, Talmadge, Kelly, Apollo Beach, FL 33572 (US); REDING, James, W., Sarasota, FL 34243 (US)
(74) Representative: Mair, Richard Douglas
(86) International application number: PCT/US2001/013765
(87) International publication number: WO 2001/082896

(56) References cited:
- WO-A-96/25915
- US-A- 2 491 416
- US-A- 2 688 586
- US-A- 2 772 999
- US-A- 3 187 747
- US-A- 4 113 851
- US-B1- 6 187 347
- DATABASE DWPI [Online] XP002944427 Retrieved from WEST Database accession no. 1991-250677 & SU 1 595 975 A (FLAX FIBER RES INST) 30 September 1990

## Description

This invention relates generally to topically applied agents for promoting blood clotting to arrest blood flow from an open wound, and more particularly to a method of applying an anhydrous hemostatic agent which may be mixed with an aqueous media just prior to its application directly over an open bleeding wound or a wound from which body fluid is flowing to accelerate flowing blood and body fluid clotting and enhance healing.

### PRIOR ART

In addition to conventional bandages, adhesive means, compresses and the like which are applied with pressure directly against a bleeding open wound, considerable effort has been directed toward the development of chemical agents in various forms which accelerate or enhance the coagulation of blood flowing from an open wound to arrest blood flow. Many of these agents are in the "clotting cascade", i.e., fibrinogen, thrombin, Factor VIII and the like. Others are based upon the use of collagens. Edwardson, in U.S. patents 5,763,411, 5,804,428, and 5,962,026, for example, teaches the use of fibrin in conjunction with a solid support in the '411 patent, and as an enzyme free sealant in the '428 patent, and as a solid hemostatic agent substantially free of catalytic enzymes.

Three U.S. Patents invented by Martin, U.S. 5,692,302, 5,874,479 and 5,981,606, are generally directed to the use of pyruvate in combination with fatty acids and an anti-oxidant as a therapeutic wound healing hemostatic agent.

Stilwell, in U.S. Patent 5,484,913 teaches the use of calcium-modified oxidized cellulose to promote faster hemostasis. In U.S. Patent 5,474,782, Winter, et al. teaches a wound healing hemostatic agent or its salt present in a pharmaceutically acceptable carrier, the preferred embodiment being a salt of sodium. Winter provides a wound dressing with a taspine compound for promoting healing rather than clotting.

In U.S. Patent 2,163,588, Cornish teaches a wound pad having very fine fibers carrying a viscous agent and a septic for arresting and clotting blood flow. Eberl, et al., in U.S. Patent 2,688,586, teaches an improved hemostatic surgical dressing with alginic acid as a clotting agent. Masci, et al. in U.S. Patents 2,772,999 and 2,773,000 also teaches hemostatic surgical dressing including a pad and free acid cellulose glycolic acid.

A patent for another hemostatic wound dressing is taught by Shelley in U.S. patent 3,206,361 having an active agent in the form of methylaminoacetocatechol hydrochloride. Likewise, Anderson, in U.S. Patent 3,328,259, another wound dressing containing a film of cellulose glycolic acid ether is provided as the hemostatic agent.

The hemostatic agent taught by Sugitachi, et al. as disclosed in U.S. Patent 4,265,233 is blood coagulation Factor VIII plus either fibrin or thrombin. A ready-to-use bandage is taught by Altshuler in U.S. Patent 4,363,319 which also contains thrombin as an active agent, the bandage all of which is contained within a sealed package.

Invented by Lindner, et al., a wound pad which is impregnated with tissue-compatible protein such as collagen and lyophilized Factor XIII, thrombin and fibrinogen, are taught in U.S. Patent No. 4,600,574. The use of collagen as a hemostatic agent within a pad that has been freeze dried is taught by Sawyer in U.S. Patent 4,606,910.

In U.S. Patent 4,616,644, Saferstein, et al. teaches the use of an adhesive bandage with high molecular weight polyethylene oxide applied to the surface of the perforated plastic film wound release cover of the bandage to arrest blood flow from minor cuts. Yet another hemostatic agent including a carrier in the shape of a flake or fiber having thrombin and Factor XIII affixed thereto is taught by Sakamoto in U.S. Patent 4,655,211. The use of an ultra-pure, dean thrombin solution as a hemostatic agent is taught in U.S. Patent 5,525,498 invented by Boctor. Two recent patents invented by Pruss, et al., U.S. 5,643,596 and 5,645,849 both teach the use of hemostatic dressings which incorporate thrombin and epsilon aminocaproic acid (EACA) and calcium chloride on gelatin.

An absorbable spun cotton-like topical hemostat is taught by Shimuzu, et al. in U.S. Patent 5,679,372. This disclosure is directed to an absorbable dressing made of acetocollagen fibers which are innately adhesive to a bleeding surface. In a patent to Bell, et al, U.S. 5,800,372, a dressing made of microfibrillar collagen and a superabsorbant polymer provides both blood absorption and clotting inducement.

WO 96/25915 discloses a dental composition comprising a) a quantity of a hemostatic agent that provides astringent action for stopping oral bleeding or providing gingival tissue fluid control, said hemostatic agent being selected from a group including ferric sulphate, ferric subsulphate and ferric chloride; b) an inorganic filler; c) a polyol; and d) an aqueous base, which composition can stop bleeding or provide gingival tissue fluid control without significantly opening up dentinal tubules in a tooth.

US Patent 2,491,416 discloses a wound dressing composed of tantalum oxide powder containing a small amount of iron oxide.

US Patent 4,113,851 discloses a composition having prolonged microbiocidal activity containing elemental iodine, a specified 2-pyrrolidone polymer, complexed with the elemental iodine, and a high molecular weight acidic activity extender, for example polycarboxylated organic acid.

US Patent 3,187,747 discloses a tampon or other fibrous absorbent dressing, which includes fibrous or filamentary material formed from a polymeric component having textile fibre-forming properties, and having integrally associated therewith a polymeric component having acidifying properties, for example a cation exchange polymer in hydrogen form.

US Patent 2,688,586 discloses a surgical dressing containing alginate material and a specified amount of acid (citric, maleic, malic, nitric, phosphoric or tartaric acids) in solution.

US Patent 2,772,999 discloses a surgical composition for coagulating blood containing as hemostatic agent a specified cellulose derivative, more particularly free acid cellulose glycolic acid ether or free acid cellulose hydroxypropionic acid ether. The hemostatic agent may also be used in bone wax compositions including a water-soluble innocuous base such as polyol compound.

The present invention provides a flowing blood or body fluid clotting agent which comprises an admixture of potassium ferrate in combination with a insoluble sulfonated cation exchange resin in the hydrogen form.

The invention further provides a hemostatic agent adapted to be applied directly onto a bleeding wound comprising:
i) an effective amount of potassium ferrate combined with an effective amount of an insoluble sulfonated cation exchange material in the hydrogen form,
   said potassium ferrate combining with blood to promote blood clotting at the wound, said cation exchange material forming a protective cover over the wound as blood is thereby clotted, or
ii) an effective amount of substantially anhydrous potassium ferrate, combined with an effective amount of an insoluble sulfonated cation exchange material in the hydrogen form, said cation exchange material being a resin which is cross-linked greater than 0.5% and has a moisture uptake which substantially exceeds 80% by weight of the total weight of said resin when fully saturated with water,
   said potassium ferrate combining with blood to promote blood clotting at the wound and said cation exchange material forming a protective cover over the wound as blood is thereby clotted.

The invention further provides a delivery vehicle for a hemostatic agent adapted to be applied to a bleeding wound, wherein said hemostatic agent comprises:-
substantially anhydrous potassium ferrate combined with an effective amount of an insoluble sulfonated cation exchange material in the hydrogen form;
said delivery vehicle taken from the group comprising:-
a fiber material impregnated with said hemostatic agent;
a powder spray carrier within a pressurized container combining with said hemostatic agent as a dry powder upon discharging of the spray carrier from a spray nozzle of the container;
a cotton swab impregnated with said hemostatic agent;
a bandage impregnated with said hemostatic agent;
a sponge impregnated with said hemostatic agent;
a porous tea bag-like enclosure containing a quantity of said hemostatic agent; and
a foam carrier within a pressurised foam container combining with said hemostatic agent upon discharge of the foam carrier from a discharge nozzle of the foam container.

In addition, the invention provides a flowing blood or body fluid clotting agent or a hemostatic agent or a delivery vehicle as specified above, or a flowing blood or body fluid clotting agent comprising an admixture of potassium ferrate with an organic acid or an acidic inorganic salt, for use in arresting the flow of blood from a bleeding wound, for healing bed sores or decubitus ulcers, for treating skin or tissue burns, or as a surgical sealant.

The present invention also provides substantially anhydrous potassium ferrate capable of hydrating in the presence of blood or an aqueous media to produce polyvalent metal ions with blood flowing from a wound, for use in a method of arresting the flow of blood from a bleeding wound, in healing bed sores or decubitus ulcers, in treating skin or tissue burns, or as a surgical sealant, said method for arresting the flow of blood from a bleeding wound comprising the steps of:-
A) mixing the substantially anhydrous potassium ferrate with said blood or an aqueous media, and
B) applying the mixture from Step A to the wound for a time sufficient to effect sufficient clotting of the blood to arrest substantial further blood flow from the wound.

The present invention further provides substantially anhydrous potassium ferrate capable of hydrating in the presence of blood to produce Fe⁺⁺⁺, for use in a method of arresting the flow of blood and other body fluids containing protein from an open skin wound, in healing bed sores or decubitus ulcers, in treating skin or tissue burns, or as a surgical sealant, said method for arresting the flow of blood and other body fluids containing protein from an open skin wound comprising the steps of:
A) mixing the substantially anhydrous potassium ferrate with a quantity of an aqueous media to form a paste;
B) applying said paste to the wound for a time sufficient to effect sufficient clotting of the blood to arrest substantial further blood or body fluid flow from the wound.

The present invention further provides substantially anhydrous potassium ferrate, and a hydrophilic proton donor which will hydrate in the presence of blood to thereby promote clotting of the blood, for use in arresting the flow of blood from a bleeding wound, in healing bed sores or decubitus ulcers, in treating skin or tissue burns, or as a surgical sealant.

The invention further provides a flowing blood or body fluid clotting agent or hemostatic agent or delivery vehicle or substantially anhydrous potassium ferrate as specified for use above, for the manufacture of a medicament for use as specified above.

One embodiment of the present invention utilizes an improved ion exchange resin, preferably in the form of a styrene divinylbenzene copolymer which has been sulfonated. The collective teaching of making this prior art resin is to be found in an earlier patent to co-inventor, Patterson, U.S. 4,291,980 which was based at least in part on the production of spherical beads comprised of copolymer styrene and divinylbenzene as taught in U.S. Patents 2,366,007 and 3,463,320. This collective teaching is incorporate herein by reference. An improvement better adapting this resin to the present invention is in the form of substantially reduced cross-linking down to about 0.25%.

A primary aspect of the present invention incorporates potassium ferrate (2K₂FeO₄). The teaching of a process for producing alkali metal ferrates is taught by another co-inventor, Thompson, in U.S. Patent 4,545,974. This teaching is also incorporated herein by reference. See also U.S. Patent 6,187,347 by co-inventors herein.

It is submitted that the above-referenced prior art, either taken individually or collectively in any combination thereof fail to teach an enhanced hemostatic agent, method or carrier for a flowing blood or body fluid clotting agent which includes an admixture of the potassium ferrate which produces a trivalent Fe⁺⁺⁺ ion which reacts with the blood to accelerate coagulation and clotting of the blood. Moreover, the utilization of an enhanced insoluble cation exchange material, being a sulfonated ion exchange resin, in combination with the potassium ferrate to form the enhance hemostatic agent, additionally produces a protective matrix covering over the wound and also supplies an oxidative capacity which acts as an antibacterial, antiviral and antifungal agent. Further, the presence of selected salts neutralize hydroxide radicals as clotting occurs so as to eliminate any substantial stinging sensation.

The "protective matrix" is formed by the interaction of the components of the invention with the liquid and solid components of the blood and tissue associated with the wound or trauma. The term clot and/or clotting includes both or either the product of the natural blood clotting cascade and the protective matrix formed by the invention. A "wound" includes all traumas resulting in the egress of blood, plasma and/or lymph from vessels and/or tissues, especially through the skin. "Body fluids" include complete blood (red blood cells, white blood cells, platelets and plasma), plasma alone, lymph alone (including suspended cells) and other body fluids, excluding urine, saliva and vaginal fluid. "Blood" refers to complete blood, plasma and/or lymph. Bleeding refers to the egress of bodily fluids including blood, plasma and/or lymph. A "hemostatic agent" refers to natural and/or artificial material which brings about stoppage of blood loss.

Neither does prior art teach another broad aspect of this invention which includes a flowing blood or body fluid clotting (hemostatic) agent which includes an admixture of potassium ferrate, in combination with a cation exchange resin, an organic acid or an acidic inorganic salt, which reacts with the blood or protein in blood to accelerate coagulation and clotting of the blood. The utilization of the insoluble cation exchange material, in combination with the oxyacid salt, potassium ferrate, also produces a protective matrix covering over the wound and also supplying oxidative capacity which acts as an antibacterial, antiviral and antifungal agent. In yet another aspect, the presence of a selected hydrophilic proton donor neutralizes hydroxide radicals as clotting occurs so as to eliminate any substantial stinging sensation.

This invention is directed also to a carrier and enhanced hemostatic agent for arresting the flow of blood and other body fluid from an open wound and for promoting wound healing. The substantially anhydrous hemostatic agent of the potassium ferrate and an enhanced cation exchange resin is provided for unique use which will hydrate in the presence of blood or other protein-containing body fluids. Fe⁺⁺⁺ is produced, thereby promoting clotting of the blood and other body fluids when applied to the open wound for a time sufficient to promptly arrest substantial further flow of bodily fluids from the wound. One preferred hemostatic agent includes substantially anhydrous potassium ferrate and an enhanced sulfonated ion exchange resin as an admixture which will rapidly hydrate in the presence of blood or other aqueous media to produce Fe⁺⁺⁺, thereby promoting clotting. The resin produces a protective matrix coating over the wound for protection and promotes healing. Oxidative capacity produced during the reaction substantially reduces the level of bacteria, virus and fungus at the wound.

Another broad aspect of this invention is directed to a substantially anhydrous admixture of potassium ferrate and a hydrophilic proton donor which will hydrate in the presence of blood and body fluid to provide protons which neutralize hydroxides and promote blood clotting, for use in arresting the flow of blood from a bleeding wound, for healing bed sores or decubitus ulcers, for treating skin or tissue burns, or as a surgical sealant. A variation includes the compound containing potassium ferrate plus a non-ionic hydrophilic polymer such as carboxy methylcellulose, polyvinyl alcohol, an alginate, starch, sugar and all soluble gums. Still another embodiment includes the compound formed of potassium ferrate in combination with a hydrophilic proton donor and a solid desiccant which promotes clot formation. The enhanced cation exchange material or an admixture of alkali metal oxyacid salt potassium ferrate plus acidic inorganic salt produces a scab or protective coating over the wound for protection and enhanced healing.

One embodiment of this invention therefore relates to potassium ferrate for use as a clotting agent for arresting blood flow from an open surface wound.

One embodiment of this invention relates to potassium ferrate hemostatic agent for use in arresting blood and body fluid flow, the agent being substantially sting-free when applied onto an open wound.

In still another embodiment of this invention an anhydrous hemostatic agent utilising potassium ferrate combined with an enhanced insoluble cation exchange material is provided for mixing with an aqueous media just prior to use for arresting blood flow from an open skin wound.

The invention facilitates the preparation of a localised rapid forming protective coating or covering that has antibacterial, antifungal and antiviral properties.

Another embodiment of the invention relates to potassium ferrate for use as a blood clotting agent for arresting blood flow from an open surface wound.

A potassium ferrate hemostatic agent according to this invention can be used for arresting blood and body fluid flow, being substantially sting-free when applied onto an open wound.

One embodiment for this invention provides a composition utilizing potassium ferrate combined with an enhanced insoluble cation exchange material or an organic acidic or an inorganic salt for example to arrest blood flow from an open skin wound.

In addition to promoting blood clotting to arrest blood flow from an open wound, a composition of potassium ferrate also provides antiseptic and a protective material over the wound.

In yet a further embodiment of the invention the fluid uptake capacity of resin mixed with potassium ferrate is enhanced by use of a lower cross-linked resin and/or appropriate treatment of the resin.
Figure 1 is a schematic flow diagram of material hemostatic agent additives and delivery modes for specific applications of the invention.
Figure 2 depicts a preferred delivery mode of the invention in the form of individual sealed ampoules.
Figure 3 depicts the enhanced moisture uptake of resin as a result of boiling treatment in hydrogen peroxide (H₂O₂) prior to admixture with the salt ferrate.

### MECHANISM OF BLOOD COAGULATION

The following is offered as a brief explanation of one possible alternative mechanism which would explain the effectiveness of the present invention as described herebelow in full detail.

### ALTERNATE THEORY

### MECHANISM OF HEMOSTATIS

Blood contains both a solid and a liquid component. The liquid component is called plasma and contains a very broad variety of proteins. Among them are albumin, immunoglobulin and an assortment of proteins which participate in blood clotting. The solid components of the blood include red blood cells (erythrocytes), white blood cells (leucocytes), and platelets. Of these, only platelets participate directly in blood clotting.

When blood dots, depending upon the immediate cause, the proteins in the plasma which are involved (which are proteolytic enzymes) act in a chain reaction (i.e. protein #1 activates protein #2 which activates, in turn, protein #3, etc.). This is called the cascade mechanism of blood clotting. Simplifying the process, the last three steps are as follows:

irritation and disruption of platelets cause the release of Prothromboplastin. Calcium ions (CA⁺⁺), which are normally present in the plasma, cause the conversion of inactive Prothromboplastin into the active proteolytic enzyme, Thromboplastin. Thromboplastin, in the presence of calcium ions (Ca⁺⁺) causes the activation of Prothrombin into Thrombin. Thrombin (also a proteolytic enzyme) acts upon Fibrinogen (present In great quantity in the plasma) to remove a portion of that protein, thus converting it to Fibrin, which then actively polymerizes with itself. A simple diagram of this clotting process is shown below.

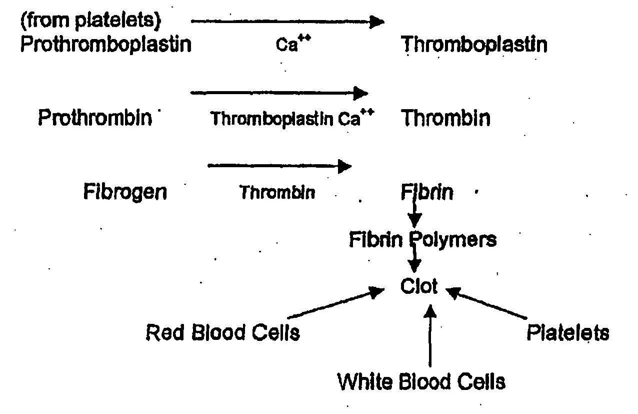

Fibrin molecules are, as the name implies, arrayed in long strands. These molecules are a very "sticky" protein causing them to adhere to the tissue surrounding the wound (both epithelial tissue and deeper laying muscle and other connective tissue). Additionally, Fibrin molecules stick not only to tissue adjacent to the wound, but also to themselves. This "self-sticking" forms a molecular web which expands and becomes stronger as more and more Fibrin strands become enmeshed across the wound. As blood continues to flow from the wound, the solid components of the blood become enmeshed, trapped and stuck upon the Fibrin "web", thus eventually blocking the egress of blood from the wound.... forming a clot.

The clot naturally falls off the damaged tissues underneath the clot (the callus of dedifferentiated wound tissue) becomes reorganized into repaired tissue.

### ALTERNATE THEORIES

### MECHANISM OF ACTION OF THE INVENTION

The invention includes a non-drug, non-biological powder for use to cover a wound to control bleeding and fluid loss, absorbs wound exudates and protects against abrasion, friction, desiccation and contamination. This powder is composed of a uniform admixture of a hydrophilic polymer and an inorganic Ferrate ionic material (potassium ferrate). In one delivery mode, the granules/powder are used by sprinkling directly onto the wound site or in the nose by using a moist cotton/Dacron applicator containing the admixture. The admixture and its constituents are applied topically and is not metabolized by the body.

The mechanism of this invention is independent of the normal clotting mechanism, the clotting cascade. The invention creates a synthetic plug or barrier by providing its own means of binding with the skin, wound tissue and blood components. The invention provides for linking trivalent ions with tissue and blood components to the resin, rapidly forming a matrix regardless of anticoagulant usage. Being independent of the normal blood clotting mechanism, it may be functional for use by persons using anti-coagulants which interfere with, and prevent coagulation and by persons afflicted with genetic defects in the blood clotting mechanism such as haemophilia. It should be noted that normal blood clotting (unless inhibited by patient therapies or prevented by genetic defects) can and does occur beneath, and simultaneously with, the invention's artificial matrix/clot formation.

A possible secondary mechanism may be that the trivalent ferric ions irritate platelets to release not only Prothromboplastin, but also biologicals involved with wound heating, thereby promoting the reconstrution/regeneration of the damaged tissue. Other possible include the chemical stimulus by trivalent Ferrate ions of the precursors to active coagulation components.

The trivalent ferric ionic component of the invention may bind to the skin and the tissues exposed by the open wound. As red and white blood cells and platelets are carried out of the wound in the plasma, they bind with the ferric ions and the resin to form a water-resistant barrier, an artificial clot to the escaping blood. The tissue exudates and plasma (the lymph and the liquid component of the blood, which is mostly water), are absorbed by the resin polymer. This absorption of fluid by the resin polymer causes it to swell vigorously. This swelling, in turn, occludes the wound site, thus controlling/stopping the flow of blood.

The protective matrix of the ferric ions, the water-swollen resin, natural solid blood components (fibrin plus the red cells, white cells and platelets) combine to cause the rapid formation of a protective matrix. In accomplishing this, the invention helps promote healing, protects against infection and minimizes the pain and discomfort to the wound area.

All polyvalent cations can induce the natural blood clotting cascade. It is known that the decomposition of potassium ferrate produces the finest particles of iron oxide (Fe₂O₃) available. (See U.S. Patent 4,545,974). Upon addition to water, K₂FeO₄ becomes Fe⁺⁺⁺ in the form of FeOOH, which upon drying, yields Fe₂ O₂. The FeOOH (or Fe₂ O₃ H₂O) is a solid in suspension and this ultra-fine material seems to be an ideal irritant for platelet membranes, thereby releasing the prothromboplastin that is needed to initialize the natural clotting cascade. It is possible that they may tend to rupture the platelets themselves, thereby causing a massive release of clotting factors as does the rough surface of a wound achieve the same end.

The Fe⁺⁺⁺ ion is an example of a polyvalent cation, that will induce coagulation of blood. Trivalent ions, by lowering the zeta potential of a particle in solution, allow the particles (platelets) to aggregate more easily. Platelets are small disks of cytoplasm found in the blood of mammals. After a wound is received they begin to aggregate and stick around the wound area, causing the aggregation and sticking of another cytoplasmic component, the thrombocycte. During this aggregation process, certain phospholipids from the membrane of the platelets contribute to the overall clotting process, combined with the inactive plasma enzyme, Factor XII. Mechanical abrasion of the platelet membranes is important in freeing the phospholipid component from the platelets.

### RANGE OF USEFUL SALT FERRATES

Initially, applicants have found that the utilization of potassium ferrate, again likely based upon the above-recited theory, effectively accomplishes the accelerated clotting of blood flowing from an open wound. The apparent chemical ferrate reaction with water found in blood is as follows:
1. 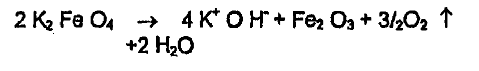
2. K O H + Fe₂ O₃ → Fe (OH)₃ ↓ + K⁺ + OH⁻
3. Fe (OH)₃ → Fe⁺⁺⁺ + 3(OH)⁻

One of the important results is the production of the trivalent Fe⁺⁺⁺ ion which appears to be the beneficial clotting agent provided in this aspect of this invention. Moreover, it has been determined that the present invention acts on all body fluids containing protein, such as that which flows from an open skin blister or bum.

In addition all zeolites, sulfonated coal, and natural occurring membranes such as protein membranes will also act in compound form with ferrate to release the trivalent Fe⁺⁺⁺ ion to effect blood and body fluid coagulation.

### ELIMINATING STINGING EFFECT

In utilizing the K₂ Fe O₄ as above described to arrest blood flow from a bleeding wound, equation 1 shows the presence of hydroxide (OH)⁻radicals which are produced. The hydroxide (OH)⁻ radicals remain present in equation 3 and cause stinging at the wound site.

In a method of arresting blood and body fluid flow from an open skin wound an effective amount of potassium ferrate, preferably in powder form, is applied directly onto the wound to interact with flowing blood or body fluid to accelerate its clotting.

### SALT FERRATE COMBINED WITH RESIN

Although the above methodology and utilization of potassium ferrate greatly enhances blood clotting, the wound nonetheless remains opened and generally unprotected unless the potassium ferrate is combined with a carrier such as a finger bandage, swab or the like which has been impregnated or coated with a dry powder taken from of one of the above chosen salt ferrate hemostatic agents.

By the addition of an ion exchange resin R with the potassium ferrate, an additional benefit of protective matrix formation or depositing of a substance produced by the reaction with water in the blood is accomplished over the open wound. Details of the hemostatic agent and method of producing the preferred ion exchange resin R in the form of styrene divinylbenzene are disclosed in the previously referenced patents and are herein incorporated by reference. As described in formulas herebelow, the resin R may be shown in its chemical form or generally designated by the symbol "R" for simplicity. The ion exchange resin R is sulfonated as is shown in chemical terms in each chemical equation herebelow.

An acid form of the sulfonated ion exchange resin R in acid form is shown symbolically as follows:

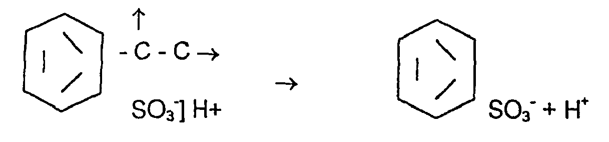

When the preferred hydrogen form of this sulfonated ion exchange resin R is in the presence of the salt ferrate and water within blood, the following reaction serves to neutralize the hydroxyl ions produced in equation 3 above.

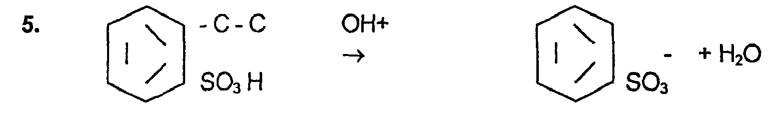

In addition to neutralizing hydroxyl ions by the presence of even trace amounts of the resin R to decrease or totally eliminate the stinging effect, excess trivalent Fe⁺⁺⁺ ions interact with the resin as follows:

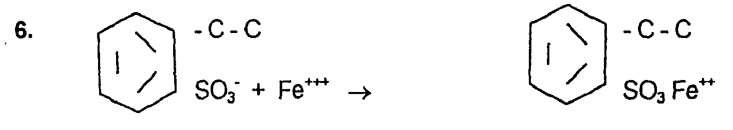

Thus, excess trivalent Fe⁺⁺⁺ charged ion cross links with the clotting blood in accordance with the following equation:

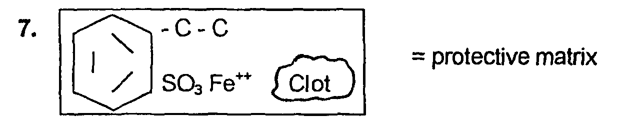

The amino acids in the blood protein are shown to interact with the resin:

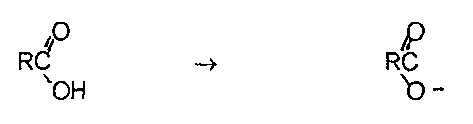

The K₂ Fe O₄ is hygroscopic, small particles approximately 50 to 100 mesh size for best surface area. The ion exchange resin R is preferably in an acid form with some substitute Ca calcium ions as shown in equations 4 to 7. The cross linking of the resin R should preferably be below 4.0 and as low as 0.25% and hygroscopic. The weight ratio should preferably favour the dry ion exchange resin R by at least 4 to 1 of dry salt ferrate. The ion exchange resin R is a cation exchange resin.

In another embodiment, a small amount of divalent calcium Ca++ may be added as an additional coagulant. Heparins, EDTA (Ethylene Dismine Tetracacitic Acid), potassium oxalate, and warfarins are anticoagulants and are ionic in nature and remove Calcium Ca⁺⁺ and trivalent ion Fe⁺⁺⁺ by chelation to inhibit the natural clotting cascade. By supplying excess of polyvalent ions, the above anticoagulants and others can be overcome and clotting can occur. Also, in addition to the hydrogen form of the resin R - 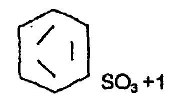 a given ratio of the calcium salt 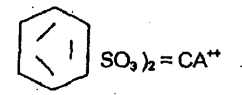 can supply excess of this ion to further Induce blood clotting. The ferrate in contact with the blood - water on the skin creates oxidative capacity which is a strong disinfectant to the wound.

### SUMMARY OF BENEFITS

By combining even a trace amount of the above-described sulfonated resin (RSO₃) as an admixture with potassium ferrate (K₂FeO₄), the following benefits are derived:
1. The trivalent Fe⁺⁺⁺ + 3 RSO₃ produces a protective matrix and blood flow stoppage;
2. The oxidizing capacity produced by the reaction serves as an antibacterial, antiviral and antifungal agent;
3. The clotting with resin R produces a protective matrix that acts as a protective coating for the wound;
4. Resin (RSO₃) in this admixture neutralizes hydroxyl ions to prevent stinging.

### EXAMPLE 1

### Anhydrous Powder Preparation

A ferrate - ion exchange resin admixture (moisture free) was prepared for direct application to a bleeding injury. The cation exchange resin R was prepared in the washed hydrogen form, and then dried at 110° for 24 hours and powdered in grinder to about 100 mesh.

This hemostatic agent was then applied directly to a fresh bleeding finger wound produced by a skin lancet having a penetration of 1.6 - 2.2 mm. The subject was 77 years old, skin condition non-flexible. Wound blood flow was at a rate of 0.206g/30 seconds or 0412g per minute to 0606g per minute.

When a single penetration of the skin was made and blood flow started at 0.0412 to 0.0605 g per minute, application of 5 sec. of the above resin - ferrate hemostatic agent directly to the wound dropped blood to zero as determined with a blot pickup of 0.0020g within 1.0 minute. The resin- ferrate applied was on the order of 0.0175 - 0.0170 grams, forming a hard protecting sterilized coating over the penetration injury by the time that blood flow from the wound was stopped.
DOSAGE ECONOMY
Pretreatment Blood Flow......0305g blood/30 sec. (.0605g/min)
After treatment Blood Flow......0010g blood/30 sec. (.0028g/min
Dosage......0174g of anhydrous ferrate and resin admixture was used to treat wound.

At this dosage, a 30g quantity of the hemostatic agent will provide approximately 1724 separate treatments.

### METHOD OF PREPARATION

### Just Prior to Use

The above-described anhydrous compound is preferably in the form of a combination of potassium ferrate (K₂FeO₄) and the acid form of low cross-linked ion exchange resin particles. Both of the materials are in powder form and are stored together in an anhydrous form (no water). They have been previously applied directly to body fluids, i.e. blood, in the dry form (powder). Using this technique, this dry compound is difficult for control as to location of application and stability on a wound.

Certain chemical reactions slow down the blood clotting action and the production of beneficial oxidizing capacity. By controlling the neutralization of potassium hydroxide (KOH) by the acid resin, the mixing time and application time can be controlled. An aqueous media is used to mix the K₂FeO₄ and RSO₃H. Thereafter, the mixture is spread on the wound to clot the blood and stop the bleeding. Mix time and spreading time total is about five minutes working time. The amount of aqueous media is just sufficient to form a spreadable paste when combined with the ferrate (VI) salt and resin. The lower the percentage of cross-linking of the resin, and the more resin used, the greater the amount of aqueous media needed.

The aqueous media that have been shown to provide the beneficial results of the present invention are:
(1) whole blood (from wound) and body fluids;
(2) deionized water;
(3) sodium chloride (ionic, aqueous);
(4) dissolved gelatin (i.e. 2% (aqueous);
(5) carboxyl methacel (aqueous);
(6) carbohydrate solution, i.e. sugar.

The following media controlling factors have been identified as being useful in the present method:
(1) ionic aqueous additive;
(2) viscosity;
(3) osmotic pH control (between pH 2 and 10) of the aqueous media;
(4) heat (5°C - 30°C).

### EXAMPLE 2

Compound ingredients:
0.1673 grams of anhydrous cation exchange resin RSO₃H [0.5% X-L]
.0215 g. of K₂FeO₄
1.020 g. of 2% gelatin (aqueous)

Mix the resin and the K₂FeO₄. Then add the gelatin and mix. Within five (5) minutes, spread this paste-like mixture on the bleeding wound. The resin ferrate applied in this form controls bleeding.

Compound ingredients:
0.1400 gm RSO₃ [2.0%X-L]
0.0120 gm

Mix the resin and K₂FeO₄ with blood from a victim's wound and then apply this paste-like mixture to the wound from which the blood was previously obtained. Cover the wound evenly with the prepared paste mixture to control the bleeding.

### ACCELERATED BLOOD CLOTTING

The present invention, in one aspect, uses potassium ferrate (an inorganic acid containing oxygen known as an oxyacid, in the salt form) in combinations as described herebelow, which appear to have a similar beneficial effect upon accelerating the coagulation of blood and other protein based fluids flowing from an open wound.

Other oxyacid salts which have been shown to produce this blood coagulation acceleration are as follows:-
1. Alkali & alkaline earth salts;
2. Oxyacid salts of transition metals;
3. Halogen oxyacids;
4. Alkali & alkaline oxides, peroxides and superoxides.

### EUMINATION OF STINGING

A hydrophilic proton donor may also be added which chemically combines to eliminate the sting caused by the presence of hydroxyl ions produced after the blood clotting reaction is in progress. In general, there are three categories of hydrophilic proton donors which will act as a matrix to accomplish the neutralization of the hydroxyl ions, where present, as follows:
1. Cation exchange resin (sulfonated, phosphorated or carbonated)
2. Acid producing salts
3. Organic acids.

Following are more specific examples of each of the three above-referenced general categories of compounds which will neutralize the hydroxyl acids present in the blood coagulation reaction of the present invention as follows:
1. Hydrogen form cation exchange resins (sulfonates)
2. Hydrogen form cation exchange resins (phosphonates)
3. Hydrogen form cation exchange resins (carbonates)
4. Acidic inorganic salts (e.g. NaHSO4)
5. Organic acids (e.g. Citric acid, carboxylic acids, amino adds, peptides, proteins)
6. Solid desiccants (e.g. CaCl2, CaSO₄)
7. Porous hydrophilic matrix resins
8. Silicates (e.g. bentonite clay, hydroxy apatite)
9. Three component oxyacid, proton donor, solid desiccant
10. Polyvinyl alcohol
11. Carboxy methylcellulose

Solid desiccants also accelerate blood dotting further by water absorption from the blood.

### PROTECTIVE MATRIX FORMATION

Another preferred function of the present invention is to create an artificial scab atop the open wound as the blood is dotted to arrest blood flow while also serving as a potential anti-microbial agent in the form of an oxidant Such artificial scab forming agents fall into two general categories. The first category is that of a cation exchange material in combination with:
1. K₂FeO₄; or
2. K₂FeO₄ + KMnO₄.

The second category is exemplified by the compound formed as an admixture of:-

NaHSO₄ + K₂FeO₄

as an unique combination of an acidic inorganic salt and an oxyacid salt respectively, also provide this artificial scab-forming agent function.

The transition metal oxyacid salts form metal oxides which are important in the matrix formation, or scab formation, when combined with the cation exchange material or any other hydrophilic proton donor. Halogen oxyacid salts do not possess this quality, nor do alkali or alkaline oxides, peroxides or superoxides. Although this later group does create an oxidizing environment that facilitates clotting, they do not act as efficiently as do the transition metal oxyacid salts to form a protective scab over the wound.

### MODES OF DELIVERY AND APPLICATIONS

Modes of product delivery and applications include the following:

Medical applications include a variety of hemostasis usages for arresting blood flow caused by minor wounds and potentially more serious wounds such as gunshots, stabs, or other severe lacerations creating blood loss. The material is expected to assist in stabilizing blood pressure and to allow the patient to maintain minimal blood loss in the most rapid manner possible.

### A. Fine powder spray system.

Inert propellants such as nitrogen, nitrous oxide and/or carbon dioxide may be utilized to optimize product performance, stability and extended shelf life. The spray is highly controlled so that the operator can direct the spray and apply desired amount of product until satisfactory wound healing occurs. Canister size would be attractive and compact to fit in small EMT equipment container or into a small pocket. For example; the significant cost benefit of the spray embodiment will allow for larger use of the product. Military personnel equipped with such a spray will be able to keep a can of material for use in emergency situations. The smaller the size, the more attractive and mobile it would become, thereby achieving larger market appeal.

### B. Bandage wound dressing.

Such delivery systems would contain the subject material in an impregnated form. The base material might be a biocompatible material such as natural or man-made material such as Dacron or cellulose that would provide additional tensile strength to an open wound. This added benefit could allow for more severe wounds to be treated. The impregnated bandage would need to be packaged in a way such that the material was not subjected to moisture in open air until immediately prior to use, maximizing effectiveness. Bandages of different sizes can be created to accommodate various size wounds.

### C. Impregnated Sponge

Application of the hemostatic agent into a sponge or sponge-like material will enable the sponge to be easily manipulated within the body cavity during surgical operations offers distinct advantages. This embodiment would maintain the advantages of arresting blood flow from sources within the body in a structure that would retain its shape and create a barrier between the source of blood flow and the surgery area. The impregnated sponge would also absorb excess blood.

### D. Tea Bag Form

The hemostatic agent can also be loaded into porous "tea bags" to achieve the maximum absorbency effect for blood or other body fluids inside the body. This concept can be applied to feminine hygiene products or the like.

### E. Foam

The use of a foam has the benefit of conforming to the exact geometry that the body cavity or wound has created. The direct benefit of the hemostatic agent reacting immediately by direct application of a foam upon contact with blood and body fluids flowing from a wound requires a specialized applicator design such that the active powder and foam is co-applied simultaneously upon demand. The container may have two separate chambers that house the foam material and the dry hemostatic agent independently to prevent activaton prior to use.

### F. Pouring and Sprinkling

Direct dispensing of the hemostatic agent directly onto the wound will arrest blood flow and produce a dot.

### G. Cotton Swab Applicator

This carrier embodiment consists of a bottle containing the dry coagulating agent into which a cotton swab could be dipped and then applied to smaller localized wound areas. Alternatively, powder may be poured onto gauze and the swab rolled in the powder. The use of the material for arresting nose bleeds could be in the form of a cotton swap applicator or the like which may have been moistened and previously been dipped into the material which is then applied directly to the nasal cavity. This should prevent further damage to the sensitive membranes within the nose that is caused by the use of other products such as cauterization.

### H. Direct Container Application

Another method of applying the invention is via a round plastic dispenser that fits snugly into a bleeding nose, the container filled with the hemostatic agent or dipped into the material immediately prior to application.

### SPECIALIZED USES

The present invention is suitable for healing bed sores or decubitus ulcers by creating a protective surface barrier or temporary skin that is flexible while producing oxidative capacity at the surface of the wound, which in turn promotes healing. These ulcers sometimes have extreme difficulty in healing because of the lack of blood flow (especially in cases of diabetics) in the wound area. The product should stimulate wound recovery by providing oxidative capacity and killing bacteria as well as providing protection from contaminated air and other aerosolized or suspended infection carriers.

The present invention is also suitable for the treatment of skin and tissue bums through the creation of a protective surface or crust over the bum area and promoting natural tissue healing by producing oxidative capacity under the damaged tissue area.

Another use includes surgical sealants for mating and/or keeping trauma-separated surfaces together. This invention could be also used in conjunction with surgical staples and other such devices and with femoral artery plug systems useful during cardiac catheterization. Ferrate promotes tissue healing and growth by providing oxidative capacity under the surface of the hard clot that is created upon contact.

A further advantage of the combined ferrate and resin product is that of meaningful fluid absorbency upon contact with resin which expands as fluid is absorbed, further acting as a plug inside of a wound. This addition of pressure further enhances the ability to arrest the flow of blood. The crosslinking of the resin polymer, which is an insoluble acid, could also be changed to control the clot matrix and structure allowing for control of clot flexibility and rigidity which is useful for applying to the surface of a wound.

Fibers such as cellulose acetate, rayon, dacron, cotton, silk and the like may be included within the material hemostatic agent to provide additional structure. This would allow for further strength and/or flexibility of the clot matrix.

Use of the invention in conjunction with repair surgery systems would also stop blood flow around and in between surgical sutures and with respect to other similar surgical procedures. The hemostatic material is perfectly suited for specialty wound dressings and wound management systems.

Veterinary use would include applying this material directly to an animal wound promoting rapid healing. It offers significantly greater protection in preventing a wound site from opening-up again as the animal moves around. This is due to the material's bonding and related adhesive characteristics.

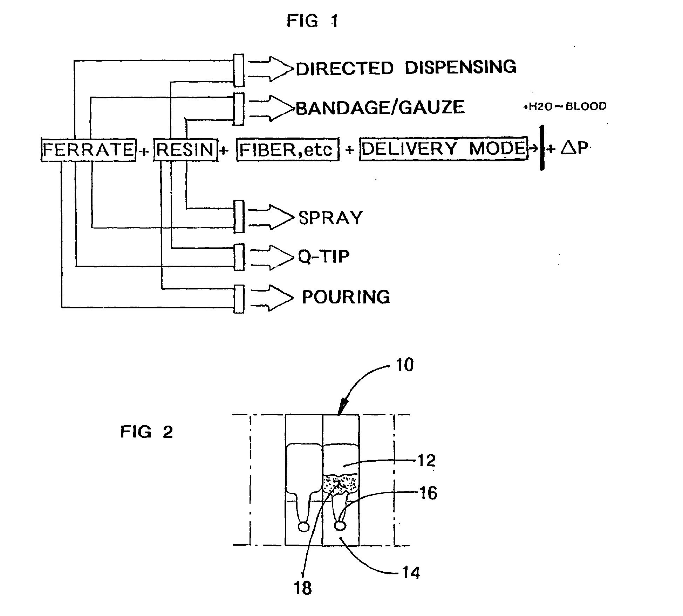

Figure 1 is a general flow diagram of the various embodiments of the modes of delivery of the hemostatic agent, including potential additives that would be complimentary for specific applications in final product form.
Figure 2 depicts an ampule-form of dispenser **10** and includes a molded plastic sealed hollow cavity **12** containing a quantity of dry hemostatic agent **18** in powder form. By separating one of the ampules dispensers **10** and fracturing and separating the removable end **14,** an opening **16** is formed from which the powder **18** may be dispensed.

### RESIN ENHANCEMENT

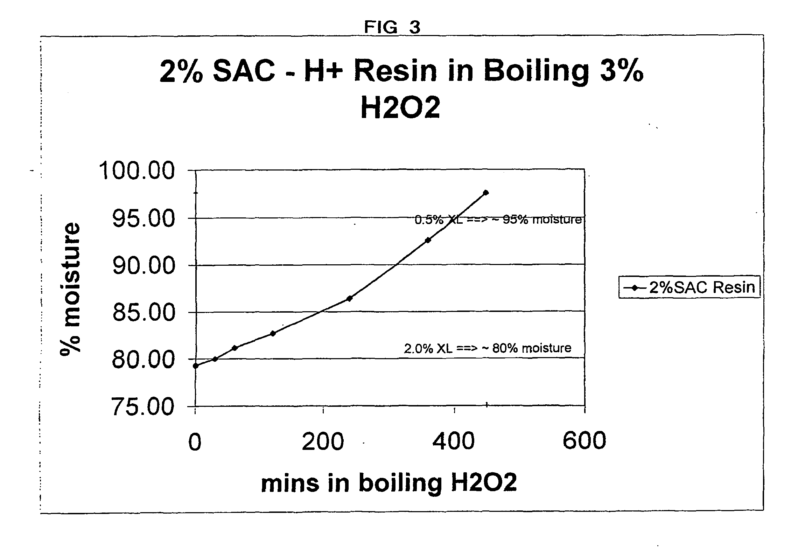

Referring now to Figure 3, the enhancement of the resin in the form of substantially increased fluid uptake will now be described in detail. As seen in Figure 3, a typical strong acid cation resin (SAC) cross-linked at a nominal 2.0% exhibits a moisture uptake capacity of approximately 80% prior to any enhancement. A 0.5% cross-link SAC resin exhibits a 95% moisture uptake capacity. These percentages are in terms of total weight of a fully water-saturated quantity of the resin. In other terms, at 80% saturation, the resin itself would represent 20% of the total weight, a weight ratio of 4:1 water-to-resin.

Because the fluid uptake or absorption of the resin is an extremely important aspect of this invention, increasing that moisture uptake capacity of the resin is highly desirable in accelerating the clotting action of the invention. To accomplish this enhancement, the resin is boiled in a time concentration of H₂O₂ (hydrogen peroxide) for a time period of up to approximately 8 hours. After boiling, the resin is filtered, washed and dried, and then infused in an admixture process with the selected salt ferrate or oxy acid salt.

The process of enhancement with respect to the 2% cross-linked resin maintains the original spherical form of the bead; however, the original spherical form of the 0.5 cross-linked bead substantially deteriorates and looses its ability to absorb fluid almost entirely. Moreover, the manufacturing economy of 2% vs. 0.5% cross-linked resin is substantial in favor of 2% cross-linking and needs less waer to effect neutrolization fo the HOH⁻ radicals described hereabove.

The enhancement process may be viewed as breaking free radicals from the resin polymer which may also be accomplished by high energy light, sonic exposure, radiation and the application of immersion in bleach. A flow diagram of the process is shown herebeleow.

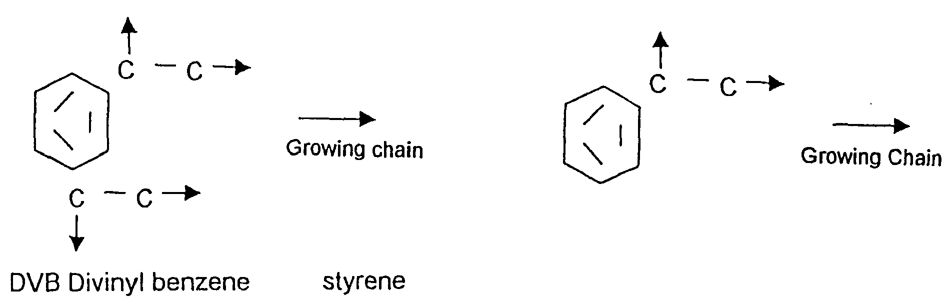

Shown diagramatically, the process of resin enhancement is shown herebelow:

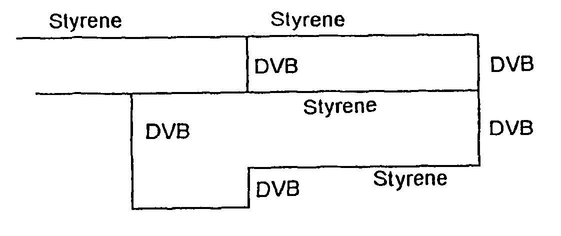

The benefit of this enhancement of the resin for increased moisture or fluid absorption is shown in Figure C. This benefit increases steadily with the time for boiling the resin in the heated hydrogen peroxide, increasing up to a percentage of moisture uptake of approximately 98% following boiling for approximately 450 minutes with lesser levels of enhancement achieved with shorter boiling times as shown in Figure 3.

While the instant invention has been shown and described herein in what are conceived to be the most practical and preferred embodiments, it is recognized that departures may be made therefrom within the scope of the invention, which is therefore not to be limited to the details disclosed herein, but is to be afforded the full scope of the claims so as to embrace any and all equivalent apparatus and articles.

## Claims

1. A hemostatic agent adapted to be applied directly onto a bleeding wound comprising
(i) an effective amount of potassium ferrate combined with a effective amount of an insoluble sulfonated cation exchange material in hydrogen form,
said potassium ferrate combining with blood to promote blood clotting at the wound, said cation exchange material forming a protective cover over the wound as blood is thereby clotted, or
(ii) an effective amount of substantially anhydrous potassium ferrate compound, combined with an effective amount of an insoluble sulfonated cation exchange material in the hydrogen form, said cation exchange material being a resin which is cross-linked greater than 0.5% and has a moisture uptake which substantially exceeds 80% by weight of the total weight of said resin when fully saturated with water,
said potassium ferrate combining with blood to promote blood clotting at the wound and said cation exchange material forming a protective cover over the wound as blood is thereby clotted.

2. A hemostatic agent as claimed in claim 1, wherein said hemostatic agent i) includes:
NaHSO₄ as an acidic inorganic salt.

3. A delivery vehicle for a hemostatic agent adapted to be applied to a bleeding wound, wherein said hemostatic agent comprises
substantially anhydrous potassium ferrate combined with an effective amount of an insoluble sulfonated cation exchange material in the hydrogen form;
said delivery vehicle taken from the group comprising:-
a fiber material impregnated with said hemostatic agent;
a powder spray carrier within a pressurized container combining with said hemostatic agent as a dry powder upon discharging of the spray carrier from a spray nozzle of the container;
a cotton swab impregnated with said hemostatic agent;
a bandage impregnated with said hemostatic agent;
a sponge impregnated with said hemostatic agent;
a porous tea bag-like enclosure containing a quantity of said hemostatic agent; and
a foam carrier within a pressurised foam container combining with said hemostatic agent upon discharge of the foam carrier from a discharge nozzle of the foam container.

4. A flowing blood or body fluid clotting agent which comprises an admixture of potassium ferrate in combination with a insoluble sulfonated cation exchange resin in the hydrogen form.

5. A flowing blood or body fluid clotting agent as claimed in claim 4, which includes a hydrophilic proton donor.

6. A flowing blood or body fluid clotting agent as claimed in claim 4, wherein the cation exchange resin is in the form of a sulfonated divinylbenzene copolymer in the hydrogen form,

7. A hemostatic agent as claimed in claim 1 or claim 2, or a delivery vehicle as claimed in claim 3, or a flowing blood or body fluid clotting agent as claimed in any one of claims 4 to 6, or a flowing blood or body fluid clotting agent comprising an admixture of potassium ferrate with an organic acid or an acidic inorganic salt, for use in arresting the flow of blood from a bleeding wound, in healing bed sores or decubitus ulcers, in treating skin or tissue burns, or as a surgical sealant.

8. Substantially anhydrous potassium ferrate capable of hydrating in the presence of blood or an aqueous media to produce polyvalent metal ions with blood flowing from a wound, for use in a method of arresting the flow of blood from a bleeding wound, in healing bed sores or decubitus ulcers, in treating skin or tissue burns, or as a surgical sealant, said method for arresting the flow of blood from a bleeding wound comprising the steps of:-
A) mixing the substantially anhydrous potassium ferrate with said blood or an aqueous media, and
B) applying the mixture from Step A to the wound for a time sufficient to effect sufficient clotting of the blood to arrest substantial further blood flow from the wound.

9. Substantially anhydrous potassium ferrate capable of hydrating in the presence of blood to produce Fe⁺⁺⁺, for use in a method of arresting the flow of blood and other body fluids containing protein from an open skin wound, in healing bed sores or decubitus ulcers, in treating skin or tissue burns, or as a surgical sealant, said method for arresting the flow of blood and other body fluids containing protein from an open skin wound comprising the steps of:
A) mixing the substantially anhydrous potassium ferrate with a quantity of an aqueous media to form a paste;
B) applying said paste to the wound for a time sufficient to effect sufficient clotting of the blood to arrest substantial further blood or body fluid flow from the wound.

10. Substantially anhydrous potassium ferrate, and a hydrophilic proton donor which will hydrate in the presence of blood to thereby promote clotting of the blood, for use in arresting the flow of blood from a bleeding wound, in healing bed sores or decubitus ulcers, in treating skin or tissue burns, or as a surgical sealant.

11. Use of
(I) A hemostatic agent as claimed in claim 1 or claim 2,
(II) a delivery vehicle as claimed in claim 3,
(III) a flowing blood or body fluid clotting agent as claimed in any one of claims 4 to 6, or
(IV) a flowing blood or body fluid clotting agent comprising an admixture of potassium ferrate with an organic acid or an acidic inorganic salt,
for the manufacture of a medicament for use in arresting the flow of blood from a bleeding wound, in healing bed sores or decubitus ulcers, in treating skin or tissue burns, or as a surgical sealant.

12. Use of substantially anhydrous potassium ferrate capable of hydrating in the presence of blood or an aqueous media to produce polyvalent metal ions with blood flowing from a wound, for the manufacture of a medicament for use in a method of arresting the flow of blood from a bleeding wound, in healing bed sores or decubitus ulcers, in treating skin or tissue burns, or as a surgical sealant said method for arresting the flow of blood from a bleeding wound comprising the steps of:-
A) mixing the substantially anhydrous potassium ferrate with said blood or an aqueous media, and
B) applying the mixture from Step A to the wound for a time sufficient to effect sufficient clotting of the blood to arrest substantial further blood flow from the wound.

13. Use of substantially anhydrous potassium ferrate capable of hydrating in the presence of blood to produce Fe⁺⁺⁺, for the manufacture of a medicament for use in a method of arresting the flow of blood and other body fluids from an open skin wound, in healing bed sores or decubitus ulcers, in treating skin or tissue burns, or as a surgical sealant, said method for arresting the flow of blood and other body fluids containing protein from an open skin wound comprising the steps of:
A) mixing the substantially anhydrous potassium ferrate with a quantity of an aqueous media to form a paste;
B) applying said paste to the wound for a time sufficient to effect sufficient clotting of the blood to arrest substantial further blood or body fluid flow from the wound.

14. Use of substantially anhydrous potassium ferrate, and a hydrophilic proton donor which will hydrate in the presence of blood to thereby promote clotting of the blood, for the manufacture of a medicament for use in a method of arresting the flow of blood from a bleeding wound, in healing bed sores or decubitus ulcers, in treating skin or tissue burns, or as a surgical sealant.

## Patentansprüche

1. Hämostatikum, das angepasst ist, um direkt auf eine blutende Wunde aufgetragen zu werden, umfassend:
(i) eine wirksame Menge an Kaliumferrat kombiniert mit einer wirksamen Menge eines unlöslichen sulfonierten Kationenaustauschmaterials in Wasserstoffform,
wobei ein Kombinieren des Kaliumferrats mit Blut eine Blutgerinnung an der Wunde begünstigt, das Kationenaustauschmaterial eine Schutzabdeckung über der Wunde bildet, wenn Blut **dadurch** geronnen wird, oder
(ii) eine wirksame Menge einer im Wesentlichen wasserfreien Kaliumferrat-Verbindung kombiniert mit einer wirksamen Menge eines unlöslichen sulfonierten Kationenaustauschmaterials in Wasserstoffform, wobei das Kationenaustauschmaterial ein Harz ist, das mehr als 0,5 % vernetzt ist und eine Feuchtigkeitsaufnahme hat, die 80 Gewichts-% des Gesamtgewichts des Harzes wesentlich übersteigt, wenn es vollständig mit Wasser gesättigt ist,
wobei ein Kombinieren des Kaliumferrats mit Blut eine Blutgerinnung an der Wunde begünstigt und das Kationenaustauschmaterial eine schützende Abdeckung über der Wunde bildet, wenn das Blut **dadurch** gerinnt.

2. Hämostatikum, wie es in Anspruch 1 beansprucht wird, wobei das Hämostatikum i) umfasst:
NaHSO₄ als saures anorganisches Salz.

3. Abgabevehikel für ein Hämostatikum, das angepasst ist, um auf eine blutende Wunde aufgetragen zu werden, wobei das Hämostatikum umfasst:
im Wesentlichen wasserfreies Kaliumferrat kombiniert mit einer wirksamen Menge eines unlöslichen sulfonierten Kationenaustauschmaterials in der Wasserstoffform;
wobei das Abgabevehikel aus der Gruppe genommen ist, die umfasst:
ein Fasermaterial, imprägniert mit dem Hämostatikum;
einen Pulversprayträger in einem Druckbehälter, der mit dem Hämostatikum als trockenes Pulver bei Austragung des Sprayträgers aus einer Sprühdüse des Behälters kombiniert wird;
ein Wattestäbchen, imprägniert mit dem Hämostatikum;
eine Bandage, imprägniert mit dem Hämostatikum;
ein Schwamm, imprägniert mit dem Hämostatikum;
eine poröse teebeutelartige Hülle, die eine Menge des Hämostatikum enthält, und
einen Schaumträger in einem unter Druck stehenden Schaumbehälter, der mit dem Hämostatikum bei Austragung des Schaumträgers aus einer Austragungsdüse des Schaumbehälters kombiniert wird.

4. Fließendes Blut oder fließende Körperflüssigkeit gerinnendes Mittel, das ein Gemisch aus Kaliumferrat in Kombination mit einem unlöslichen sulfonierten Kationenaustauschharz in der Wasserstoffform umfasst.

5. Fließendes Blut oder fließende Körperflüssigkeit gerinnendes Mittel, wie es in Anspruch 4 beansprucht ist, welches einen hydrophilen Protonendonor umfasst.

6. Fließendes Blut oder fließende Körperflüssigkeit gerinnendes Mittel, wie es in Anspruch 4 beansprucht ist, wobei das Kationenaustauschharz in der Form eines sulfonierten Divinylbenzol-Copolymers in der Wasserstoffform vorliegt.

7. Hämostatikum, wie es in Anspruch 1 oder Anspruch 2 beansprucht ist, oder ein Abgabevehikel, wie es in Anspruch 3 beansprucht ist, oder ein fließendes Blut oder fließende Körperflüssigkeit gerinnendes Mittel, wie es in einem der Ansprüche 4 bis 6 beansprucht ist, oder ein fließendes Blut oder fließende Körperflüssigkeit gerinnendes Mittel, das ein Gemisch aus Kaliumferrat mit einer organischen Säure oder einem sauren anorganischen Salz umfasst, zur Verwendung beim Stillen des Blutflusses aus einer blutenden Wunde, bei der Heilung von Liegedruckstellen oder Dekubitus-Geschwüren, bei der Behandlung von Haut- oder Gewebeverbrennungen oder als chirurgisches Dichtmittel.

8. Im Wesentlichen wasserfreies Kaliumferrat, das fähig ist, in Gegenwart von Blut oder einem wässrigen Medium unter Erzeugung von mehrwertigen Metallionen mit Blut, das aus einer Wunde fließt, zu hydratisieren, zur Verwendung in einem Verfahren zum Stillen des Blutflusses aus einer blutenden Wunde, bei der Heilung von Liegedruckstellen oder Dekubitus-Geschwüren, bei der Behandlung von Haut- oder Gewebeverbrennungen oder als chirurgisches Dichtmittel, wobei das Verfahren zum Stillen des Blutflusses aus einer blutenden Wunde die Schritte:
A) Vermischen des im Wesentlichen wasserfreien Kaliumferrats mit dem Blut oder einem wässrigen Medium und
B) Auftragen des Gemisches aus Schritt A auf die Wunde für eine ausreichende Zeit, um eine ausreichende Gerinnung des Bluts unter Stillen eines wesentlichen weiteren Blutflusses aus der Wunde zu erreichen, umfasst.

9. Im Wesentlichen wasserfreies Kaliumferrat, das fähig ist, in Gegenwart von Blut unter Herstellung von Fe⁺⁺⁺ zu hydratisieren, zur Verwendung in einem Verfahren zum Stillen des Flusses von Blut und anderen Körperflüssigkeiten, die Protein enthalten, aus einer offenen Hautwunde, bei der Heilung von Liegedruckstellen oder Dekubitus-Geschwüren, bei der Behandlung von Haut- oder Gewebeverbrennungen oder als chirurgisches Dichtmittel, wobei das Verfahren zum Stillen des Flusses von Blut und anderen Körperflüssigkeiten, die Protein enthalten, aus einer offenen Hautwunde die Schritte:
A) Vermischen des im Wesentlichen wasserfreien Kaliumferrats mit einer Menge eines wässrigen Mediums unter Bildung einer Paste;
B) Auftragen der Paste auf die Wunde für eine Zeit, die ausreichend ist, um eine ausreichende Gerinnung des Bluts zu erreichen, um wesentlichen weiteren Blut- oder Körperflüssigkeitsfluss aus der Wunde zu stillen, umfasst.

10. Im Wesentlichen wasserfreies Kaliumferrat und ein hydrophiler Protonendonor, der in Gegenwart von Blut hydratisieren wird, um **dadurch** eine Gerinnung des Bluts zu begünstigen, zur Verwendung beim Stillen des Blutflusses aus einer blutenden Wunde, bei der Heilung von Liegedruckstellen oder Dekubitus-Geschwüren, bei der Behandlung von Haut- oder Gewebeverbrennungen oder als chirurgisches Dichtmittel.

11. Verwendung
(I) eines Hämostatikums, wie es in Anspruch 1 oder Anspruch 2 beansprucht ist,
(II) eines Abgabevehikels, wie es in Anspruch 3 beansprucht ist,
(III) eines fließendes Blut oder fließende Körperflüssigkeit gerinnenden Mittels, wie es in einem der Ansprüche 4 bis 6 beansprucht ist, oder
(IV) eines fließendes Blut oder fließende Körperflüssigkeit gerinnenden Mittels, umfassend ein Gemisch von Kaliumferrat mit einer organischen Säure oder einem sauren anorganischen Salz,
für die Herstellung eines Medikaments zur Verwendung beim Stillen des Blutflusses aus einer blutenden Wunde, bei der Heilung von Liegedruckstellen oder Dekubitus-Geschwüren, bei der Behandlung von Haut- oder Gewebeverbrennungen oder als chirurgisches Dichtmittel.

12. Verwendung eines im Wesentlichen wasserfreien Kaliumferrats, das fähig ist, in Gegenwart von Blut oder eines wässrigen Mediums unter Herstellung mehrwertiger Metallionen mit Blut, das aus einer Wunde fließt, zu hydratisieren, für die Herstellung eines Medikaments zur Verwendung in einem Verfahren zum Stillen des Blutflusses aus einer blutenden Wunde, bei der Heilung von Liegedruckstellen oder Dekubitus-Geschwüren, bei der Behandlung von Haut- oder Gewebeverbrennungen oder als chirurgisches Dichtmittel, wobei das Verfahren zum Stoppen des Blutflusses aus einer blutenden Wunde die Schritte:
A) Vermischen des im Wesentlichen wasserfreien Kaliumferrats mit dem Blut oder einem wässrigen Medium und
B) Auftragen des Gemisches aus Schritt A auf die Wunde für eine ausreichende Zeit, um eine ausreichende Gerinnung des Bluts unter Stillen eines wesentlichen weiteren Blutflusses aus der Wunde zu erreichen,
umfasst.

13. Verwendung eines im Wesentlichen wasserfreien Kaliumferrats, das fähig ist, in Gegenwart von Blut unter Herstellung von Fe⁺⁺⁺ zu hydratisieren, zur Herstellung eines Medikaments zur Verwendung in einem Verfahren zum Stillen des Flusses von Blut oder anderen Körperflüssigkeiten aus einer offenen Hautwunde, bei der Heilung von Liegedruckstellen oder Dekubitus-Geschwüren, bei der Behandlung von Haut- oder Gewebeverbrennungen oder als chirurgisches Dichtmittel, wobei das Verfahren zum Stillen des Flusses von Blut und anderen Körperflüssigkeiten, die Protein enthalten, aus einer offenen Hautwunde die Schritte:
A) Vermischen des im Wesentlichen wasserfreien Kaliumferrats mit einer Menge eines wässrigen Mediums unter Bildung einer Paste und
B) Auftragen der Paste aus Schritt A auf die Wunde für eine ausreichende Zeit, um eine ausreichende Gerinnung des Bluts unter Stillen eines wesentlichen weiteren Flusses von Blut oder Körperflüssigkeit aus der Wunde zu erreichen,
umfasst.

14. Verwendung von im Wesentlichen wasserfreiem Kaliumferrat und einem hydrophilen Protonendonors, der in Gegenwart von Blut hydratisieren wird, um **dadurch** die Gerinnung des Bluts zu begünstigen, für die Herstellung eines Medikaments zur Verwendung in einem Verfahren zum Stillen des Blutflusses aus einer blutenden Wunde, bei der Heilung von Liegedruckstellen oder Dekubitus-Geschwüren, bei der Behandlung von Haut- oder Gewebeverbrennungen oder als chirurgisches Dichtmittel.

## Revendications

1. Agent hémostatique adapté pour être appliqué directement sur une plaie hémorragique comprenant :
(i) une quantité efficace de ferrate de potassium combinée à une quantité efficace d'un matériau d'échange de cations sulfoné insoluble sous forme hydrogénée ;
ledit ferrate de potassium se combinant avec le sang pour favoriser la coagulation du sang au niveau de la plaie, ledit matériau d'échange de cations formant un revêtement protecteur sur la plaie à mesure que le sang coagule de cette façon, ou
(ii) une quantité efficace du composé de ferrate de potassium sensiblement anhydre, combinée avec une quantité efficace d'un matériau d'échange de cations sulfoné insoluble sous la forme hydrogénée, ledit matériau d'échange de cations étant une résine qui est réticulée à plus de 0,5 % et qui présente une absorption d'humidité qui dépasse sensiblement 80 % en poids du poids total de ladite résine quand elle est complètement saturée d'eau,
ledit ferrate de potassium se combinant avec le sang pour favoriser la coagulation du sang au niveau de la plaie et ledit matériau d'échange de cations formant un revêtement protecteur sur la plaie à mesure que le sang coagule de cette façon.

2. Agent hémostatique selon la revendication 1, où ledit agent hémostatique i) comprend :
NaHSO₄ en tant qu'un sel inorganique acide.

3. Véhicule d'administration d'un agent hémostatique adapté pour être appliqué à une plaie hémorragique, où ledit agent hémostatique comprend
du ferrate de potassium sensiblement anhydre combiné à une quantité efficace d'un matériau d'échange de cations sulfoné insoluble sous la forme hydrogénée ;
ledit véhicule d'administration étant choisi parmi le groupe comprenant :
un matériau fibreux imprégné avec ledit agent hémostatique ;
un support de pulvérisation de poudres dans un récipient sous pression se combinant avec ledit agent hémostatique sous forme d'une poudre sèche lors de l'évacuation du support de pulvérisation depuis une buse de pulvérisation du récipient ;
un tampon en coton imprégné avec ledit agent hémostatique ;
un pansement imprégné avec ledit agent hémostatique ;
une éponge imprégnée avec ledit agent hémostatique ;
une enveloppe poreuse similaire à un sachet de thé contenant une quantité dudit agent hémostatique ; et
un support en mousse au sein d'un récipient de mousse sous pression se combinant avec ledit agent hémostatique lors de l'évacuation du récipient de mousse depuis une buse d'évacuation du récipient de mousse.

4. Agent de coagulation du sang ou d'un fluide corporel s'écoulant qui comprend un mélange de ferrate de potassium en combinaison avec une résine à échange de cations sulfonée insoluble sous la forme hydrogénée.

5. Agent de coagulation du sang ou d'un fluide corporel s'écoulant selon la revendication 4, qui comprend un donneur de protons hydrophile.

6. Agent de coagulation du sang ou d'un fluide corporel s'écoulant selon la revendication 4, où la résine à échange de cations est sous la forme d'un copolymère de divinylbenzène sulfoné sous la forme hydrogénée.

7. Agent hémostatique selon la revendication 1 ou la revendication 2, ou véhicule d'administration selon la revendication 3, ou agent de coagulation du sang ou d'un fluide corporel s'écoulant tel que revendiqué dans l'une quelconque des revendications 4 à 6, ou agent de coagulation du sang ou d'un fluide corporel s'écoulant comprenant un mélange de ferrate de potassium avec un acide organique ou un sel d'acide inorganique, destiné à être utilisé pour arrêter l'écoulement du sang d'une plaie hémorragique, pour soigner des escarres ou escarres de décubitus, pour traiter des brûlures de la peau ou des tissus, ou en tant que mastic chirurgical.

8. Ferrate de potassium sensiblement anhydre au pouvoir hydratant en présence de sang ou d'un milieu aqueux pour produire des ions métalliques multivalents avec du sang s'écoulant d'une plaie, destiné à être utilisé dans un procédé pour arrêter l'écoulement du sang d'une plaie hémorragique, pour soigner des escarres ou escarres de décubitus, pour traiter des brûlures de la peau ou des tissus, ou en tant que mastic chirurgical, ledit procédé pour arrêter l'écoulement du sang d'une plaie hémorragique comprenant les étapes consistant à :
A) mélanger le ferrate de potassium sensiblement anhydre avec du sang ou un milieu aqueux, et
B) appliquer le mélange provenant de l'étape A à la plaie pendant un temps suffisant pour réaliser une coagulation suffisante du sang pour arrêter sensiblement l'écoulement du sang de la plaie.

9. Ferrate de potassium sensiblement anhydre capable d'hydratation en présence de sang pour produire Fe⁺⁺⁺, destiné à être utilisé dans un procédé pour arrêter l'écoulement du sang et d'autres fluides corporels contenant des protéines provenant d'une plaie ouverte de la peau, pour soigner des escarres ou escarres de décubitus, pour traiter des brûlures de la peau ou des tissus, ou en tant que mastic chirurgical, ledit procédé pour arrêter l'écoulement du sang et d'autres fluides corporels contenant des protéines provenant d'une plaie ouverte de la peau comprenant les étapes consistant à :
A) mélanger le ferrate de potassium sensiblement anhydre avec une quantité d'un milieu aqueux pour former une pâte ;
B) appliquer ladite pâte à la plaie pendant un temps suffisant pour réaliser une coagulation suffisante du sang afin d'arrêter sensiblement l'écoulement du sang ou du fluide corporel de la plaie.

10. Ferrate de potassium sensiblement anhydre, et donneur de protons hydrophile qui va s'hydrater en présence du sang pour favoriser ainsi la coagulation du sang, destiné à être utilisé pour arrêter l'écoulement du sang provenant d'une plaie hémorragique, pour soigner des escarres ou escarres de décubitus, pour traiter des brûlures de la peau ou des tissus, ou en tant que mastic chirurgical.

11. Utilisation de
(I) un agent hémostatique selon la revendication 1 ou la revendication 2,
(II) un véhicule d'administration selon la revendication 3,
(III) un agent de coagulation du sang ou d'un fluide corporel s'écoulant selon l'une quelconque des revendications 4 à 6, ou
(IV) un agent de coagulation du sang ou d'un fluide corporel s'écoulant comprenant un mélange de ferrate de potassium avec un acide organique ou un sel d'acide inorganique ;
pour la préparation d'un médicament destiné à être utilisé pour arrêter l'écoulement du sang provenant d'une plaie hémorragique, pour soigner des escarres ou escarres de décubitus, pour traiter des brûlures de la peau ou des tissus, ou en tant que mastic chirurgical.

12. Utilisation de ferrate de potassium sensiblement anhydre capable d'hydratation en présence de sang ou d'un milieu aqueux pour produire des ions métalliques multivalents avec du sang s'écoulant d'une plaie, pour la préparation d'un médicament destiné à être utilisé dans un procédé d'arrêt de l'écoulement du sang provenant d'une blessure hémorragique, pour soigner des escarres ou escarres de décubitus, pour traiter des brûlures de la peau ou des tissus, ou en tant que mastic chirurgical, ledit procédé pour arrêter l'écoulement du sang provenant d'une plaie hémorragique comprenant les étapes consistant à :
A) mélanger le ferrate de potassium sensiblement anhydre avec ledit sang ou un milieu aqueux, et
B) appliquer le mélange provenant de l'étape A à la plaie pendant un temps suffisant pour réaliser une coagulation suffisante du sang afin d'arrêter sensiblement l'écoulement du sang provenant de la plaie.

13. Utilisation de ferrate de potassium sensiblement anhydre capable d'hydratation en présence de sang pour produire Fe⁺⁺⁺, pour la préparation d'un médicament destiné à être utilisé dans un traitement pour arrêter l'écoulement du sang et d'autres fluides corporels provenant d'une plaie ouverte de la peau, pour soigner des escarres ou escarres de décubitus, pour traiter des brûlures de la peau ou des tissus, ou en tant que mastic chirurgical, ledit procédé pour arrêter l'écoulement du sang et d'autres fluides corporels contenant des protéines provenant d'une plaie ouverte de la peau comprenant les étapes consistant à :
A) mélanger le ferrate de potassium sensiblement anhydre avec une quantité d'un milieu aqueux pour former une pâte ;
B) appliquer ladite pâte à la plaie pendant un temps suffisant pour réaliser une coagulation suffisante du sang afin d'arrêter sensiblement l'écoulement du sang ou du fluide corporel provenant de la plaie.

14. Utilisation de ferrate de potassium sensiblement anhydre, et d'un donneur de protons hydrophile qui va s'hydrater en présence de sang et favoriser ainsi la coagulation du sang, pour la préparation d'un médicament destiné à être utilisé dans un procédé d'arrêt de l'écoulement du sang provenant d'une plaie hémorragique, pour cicatriser des escarres ou escarres de décubitus, pour traiter des brûlures de la peau ou des tissus, ou en tant que mastic chirurgical.
